# EUROPEAN PATENT SPECIFICATION

(11) **EP 0 582 959 B1**
(45) Date of publication and mention of the grant of the patent: **29.04.1998**
(21) Application number: 93112494.5
(22) Date of filing: 04.08.1993
(51) Int. Cl.: A61M 1/16

(54) **Heat exchanger in an extracorporeal blood circuit**
Wärmeaustauscher in einem extrakorporalen Blutkreislauf
Echangeur de chaleur dans un circuit sanguin extracorporel

(30) Priority: 10.08.1992 IT MI921975
(43) Date of publication of application: 16.02.1994
(73) Proprietor: DIDECO S.p.A., I-41037 Mirandola (Province of Modena) (IT)
(72) Inventor: Ghelli, Nicola, I-40018 San Pietro In Casale (Bologna) (IT); Grandi, Giuseppe, I-41038 San Felice Sul Panaro (Modena) (IT); Panzani, Ivo, I-41037 Mirandola (Modena) (IT)
(74) Representative: Modiano, Guido, Dr.-Ing.

(56) References cited:
- EP-A- 0 089 748
- EP-A- 0 114 732
- WO-A-91/06327
- FR-A- 2 091 563
- US-A- 4 374 088

## Description

The present invention relates to a heat exchanger for an extracorporeal blood circuit.

It is known that extracorporeal blood circuits are created during surgery. The circuits typically include a heat exchanger in which the blood exchanges heat with a fluid (usually water) to regulate blood temperature, and a mass transfer device such as an oxygenation apparatus which supplies oxygen to the blood. Frequently, the oxygenation apparatus and the heat exchanger are combined in a single device.

A common embodiment of known heat exchangers provides for the flow of blood and water in respective contiguous interspaces or chambers. Also, exchangers are known in which one wall of the chamber or interspace is corrugated, whereas the other one is smooth. An example of such a heat exchanger is one in which the blood circuit is provided in a single-use module which is removably associated with a water distributor or manifold designed for continued use.

The above-described configuration leads to disadvantageous performance, since the blood undergoes heavy load losses, i.e., a pressure differential between the blood entering and exiting the device, with the danger of damaging the blood. In order to reduce these load losses it is necessary to resort to configurations which increase priming, i.e., the amount of blood contained in the device. Increased priming adds further negative attributes to the heat exchanger including a reduced circulation rate of the blood through the device causing stagnation and clotting of the blood.

For these reasons, known heat exchangers are not fully satisfactory and are not suited to be reduced in size so that they can be coupled to so-called infant or neonatal oxygenation apparatuses to be used with patients having an extremely low weight, with the necessary miniaturization. In these situations the patients have a small blood supply that cannot be used to prime the heat exchanger. Therefore, a solution or donor blood must be used to prime the heat exchanger which is generally recognized as an undesirable situation. Also, prior art combinations of a heat exchanger and an oxygenation apparatus in a single device provides a bulky and often unwieldy structure.

Known from EP-A-0 114 732 is a blood oxygenator including a heat exchanger, the heat exchanger being of a coiled bellow configuration including a plurality of individual smooth rounded surface ribs spaced apart by smaller diameter sections, inner and outer scalloped members engaging opposed portions of the bellows ribs and providing a spiral blood flow path along adjacent windings of the heat exchanger coil.

An object of the present invention is to provide a heat exchanger for an extracorporeal blood circuit which exploits fluid-dynamic characteristics for improved heat exchange, in conjunction with a low priming requirement, so that the heat exchanger can be coupled advantageously to infant and neonatal oxygenation apparatuses.

A further object of the present invention is to provide a heat exchanger for an extracorporeal blood circuit which can be associated with an oxygenation apparatus in a compact system, minimizing bulk and facilitating the handling of the system.

The above objects are achieved by a heat exchanger in an extracorporeal blood circuit, according to the invention, as defined in the appended claims.

The invention relates to an improved heat exchanger which may be associated with a hollow-fiber oxygenation apparatus or other mass transfer means. The oxygenation apparatus includes a coaxial external body monolithically associated with the substantially cylindrical shell, by means of two end heads, with the interposition of an annular portion of space containing hollow fibers. The hollow fibers are embedded at the ends of the external body in rings of polyurethane resin, termed potting. The annular portion of space is connected, at one hand, to openings for the exit of the blood from the first annular chamber and, at the other end, with a blood exit connector. The first head comprises an oxygen inlet section and the second head comprises at least an oxygen outlet section.

Further characteristics and advantages will become apparent from the description of a preferred, but not exclusive, embodiment of the invention, illustrated only by way of example in the accompanying drawings, wherein:
figure 1 is a longitudinal sectional view of the heat exchanger according to the invention wherein the module comprising the oxygenation apparatus and the blood circuit of the heat exchanger and the module comprising the manifold are associated;
figure 2 is a longitudinal sectional view of the module comprising the oxygenation apparatus and blood circuit of the heat exchanger;
figure 3 is a longitudinal sectional view of the module comprising the manifold;
figure 4 is a schematic exploded view of the shell, the core, and the manifold with discordant helical corrugations which can be seen in figures 1 through 3;
figure 5 is a schematic view of the shell, the core, and the manifold according to an alternative embodiment of the present invention with concordant helical corrugations.

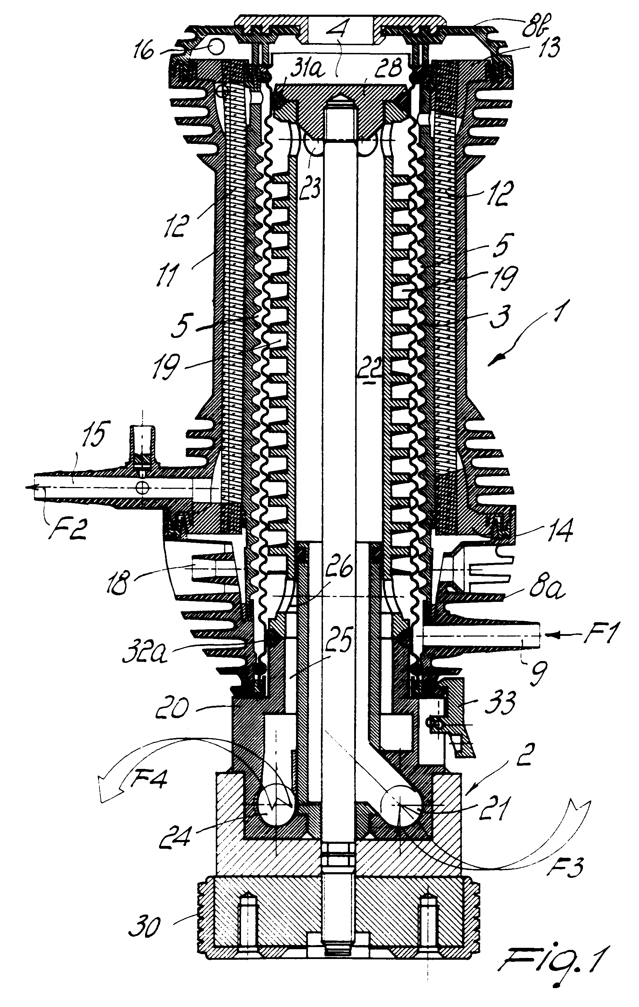

With reference to the above figures 1 to 4, the reference numerals 1 and 2, respectively, designate a first disposable module comprising the mass transfer means, typically an oxygenation apparatus, and the blood circuit of the heat exchanger and a second module comprising the water distributor or manifold of the heat exchanger. These modules, 1 and 2, when assembled together, produce the blood oxygenation apparatus with heat exchanger shown in figure 1.

In the preferred embodiment of the invention, oxygenation/heat exchanger 1 is a single-use module, whereas manifold 2 can be used repeatedly. Therefore, manifold 2 is normally connected to a fixed support, and removable oxygenation/heat exchanger modules 1 are coupled with it. Typically, an oxygenation/heat exchanger 1 is locked in a removable manner at the beginning of an operation and is removed and discarded at the end of the operation.

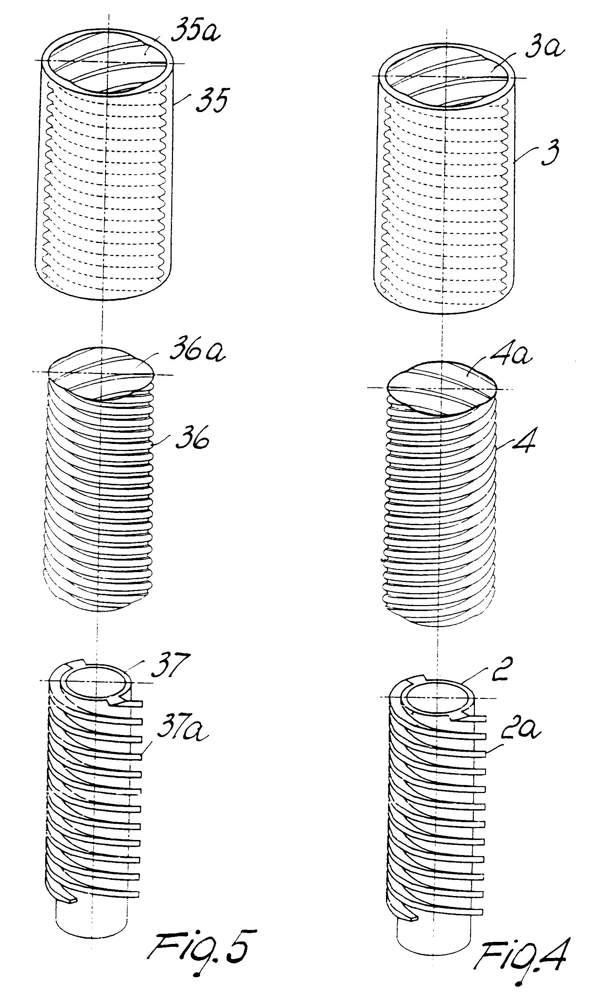

The blood circuit of integrally constructed module 1 includes a hollow and substantially cylindrical shell 3, having an interior surface with left-handed, multiple-start, helical corrugations 3a. Figure 4 shows the helical corrugations 3a having four starts.

A tube or core 4 is coaxially aligned within shell 3 and its wall is shaped so as to form right-handed, helical corrugations 4a with four starts, and is made of a material which is a good heat conductor, for example, steel. A first annular chamber 5 is thus formed between the interior surface of shell 3 and corrugated outer surface of core 4. Chamber 5 is closed by a first sealing means at its upper and lower ends. The upper end is closed by an O-ring sealing gasket 6, interposed between core 4 and shell 5. Chamber 5 is closed at its lower end by a double seal formed by O-ring gasket 7, interposed between core 4 and the internal wall of a head 8a (which will be described in detail hereinafter), and by a potted polyurethane resin ring 7a. Ring 7a is located in a channel at the lower end of head 8a and the end of core 4 is embedded therein. Ring 7a also provides stability to core 4.

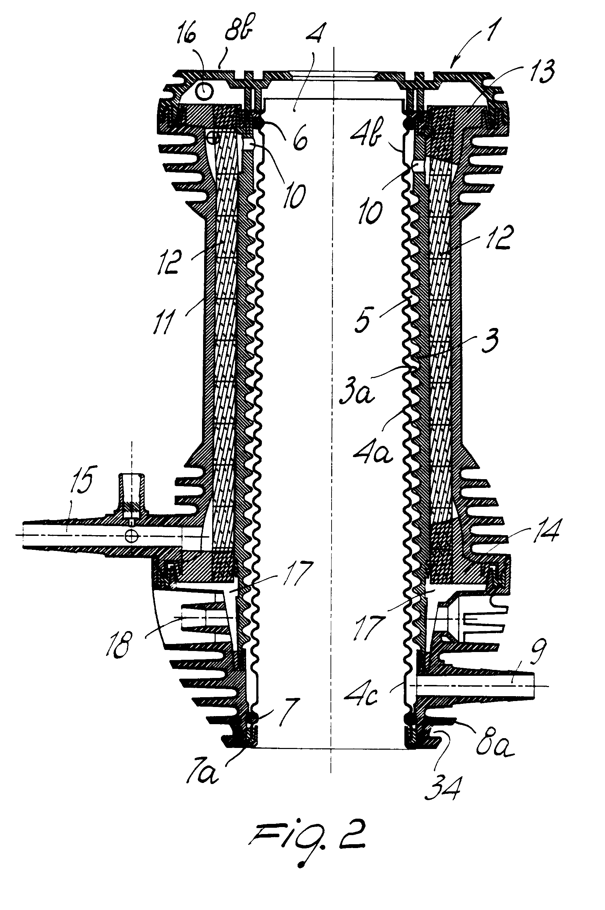

As shown in figures 1 and 2, core 4 and the internal surface of shell 3 both have slight tapers from top to bottom. During assembly, the crests or outer periphery of the corrugations of the outer surface of core 4 contact at one or more points the corrugations of the internal surface of shell 3 due to the discordant orientation of the core 4 and shell 3 corrugations. These points of contact correctly position the core 4 within the shell 3. Also, the outer periphery of the corrugations on the exterior surface of the manifold 2 may contact at one or more points the corrugations of the corrugated inner surface of core 4 due to the discordant orientation of manifold 2 and core 4.

Blood enters chamber 5 through a connector 9 located within head 8a in the direction indicated by the arrow F1 of figure 1 forming a first fluid inlet means, and leaves blood chamber 5 through openings 10, forming a first fluid outlet means. As blood travels through chamber 5, the discordant helical corrugations create an intense turbulence of the blood, preventing the formation of boundary layers, which limit heat transfer, of the blood volume passing through chamber 5.

External body 11 is monolithically associated with shell 3 by means of heads 8a and 8b. External body 11 is coaxial to shell 3. The annular portion of space between body 11 and shell 3 contains hollow fibers 12 having an inner and outer surface, which are embedded at each end of body 11 in polyurethane resin rings 13 and 14, by a process known as potting.

Blood exits chamber 5 and enters the annular portion of space of the mass transfer means through openings 10, flowing over fibers 12 and absorbing oxygen or an oxygen-containing gas. As oxygen flows through fibers 12, some of the oxygen passes through the microporous membrane which constitutes the wall of fibers 12 and is absorbed into the blood flowing over fibers 12. The blood leaves the annular portion of space through connector 15 which is monolithically formed in body 11, along the direction of the arrow F2 of figure 1.

Oxygen is supplied to hollow fibers 12 through hole 16 formed within head 8b. The part of the oxygen which does not pass into the blood through the walls of fibers 12 reaches section 17, which is provided with output connector 18.

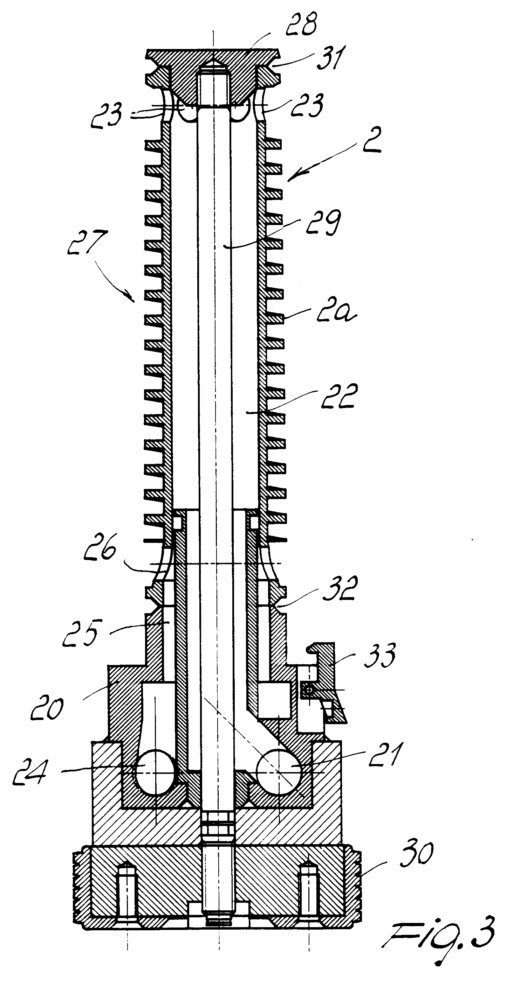

The manifold 2 of the device is now described with particular reference to figure 3. Manifold 2 is inserted in the internal cavity of module 1 , i.e., coaxially aligned within core 4, as shown in figure 1. The external surface of manifold 2 is provided with left-handed, two-start, helical corrugations 2a. A second annular chamber 19 is defined by the helical corrugated exterior surface of manifold 2 and the corrugated inner surface of core 4. As water flows through chamber 19, the discordant helical corrugations 2a (left-handed) and 4a (right-handed) create an intense turbulence in the water flow, preventing the formation of boundary layers, which limit heat transfer, for the water volume passing through water chamber 19.

At the lower end of manifold 2, base 20 includes connector 21 for water entry along the direction indicated by the arrow F3. Connector 21 is connected to inlet duct 22 which extends axially inside manifold 2 and reaches the upper end, where openings 23 connect duct 22 with water chamber 19, all forming a second fluid inlet means.

Base 20 also includes connector 24 for water discharge along the direction indicated by the arrow F4. Connector 24 is connected to duct 25, which is coaxial to duct 22. Openings 26 connect duct 25 with water chamber 19, forming a second fluid outlet means. Therefore, water entering from connector 21 passes through duct 22 in an upward direction, then passes, by means of openings 23, into water chamber 19 and flows downward through chamber 19, passing along the inner surface of tube 4 in the opposite direction of the blood which flows through chamber 5 in an upward direction and passing along the outer surface of tube 4, effectively exchanging heat. Finally, the water enters duct 25 by means of openings 26, and flows into the outlet connector 24.

Water chamber 19 is closed at each end by a second sealing means including gaskets which must be able to expand to provide watertightness during the operation of the device and must contract when a module 1 is inserted or removed. For this purpose, manifold 2 includes, adjacent to base 20, section 27 on which washer 28 is superimposed. Washer 28 is shaped complementarily to stem 29 which is threaded at its end and extends from knob 30. Section 27 includes the external surface with helical corrugations 2a, openings 23 and openings 26.

O-ring gaskets 31a, 32a are provided in seats 31 and 32, respectively, which are formed between section 27 and washer 28 and between section 27 and base 20, respectively. Knob 30 can be activated to compress gasket 31a, 32a to cause expansion in contact with the circumferential bands, respectively, 4b and 4c, of core 4 when the device is to be used and watertightness is essential, or can be released in order to remove module 1.

It should be noted that the provision of independent gaskets for blood chamber 5 and water chamber 19 ensures that any leakage of one of the gaskets does not cause the mixing of the blood and water, and fluid (blood or water) simply leaks outward.

As shown in figure 1, a removable locking means allows for the selective removal or attachment of disposable module 1 to manifold 2. Base 20 includes a plurality of pivoting claws 33 which are manually activated to release from or lock into groove 34 in head 8a. Alternatively, a spring mechanism could be used to activate claws 33.

The present invention incorporates the fluid-dynamic advantages of the helically corrugated chamber walls, effectuating an improved heat transfer characteristic. Further, the present invention reduces the need for priming which allows for a compact, easy-to-handle device that is desirable in the operating room environment.

The present invention is susceptible to numerous modifications and variations, all of which are within the scope of the inventive concept. Thus, for example, figure 5 is a view of a shell 35, core 36, and manifold 37 which have helical corrugations, respectively 35a, 36a, 37a, which have concordant left-handed orientations. In other embodiments, for example, the water distributor 2 and core corrugations 4a are left-handed whereas the shell corrugations 3a are right-handed.

It is furthermore obvious that the number of starts of the helical corrugations may vary. The same is true for the method of providing the seals at the ends of the blood and water chambers.

In the preferred embodiment, core 4 and interior surface of the shell 3 have a slight taper. According to another embodiment, both core 4 and shell surface may be perfectly cylindrical, and in this case appropriate references for correct mutual assembly are provided. Further, other fluids with good heat exchange characteristics can be used to replace water.

Finally, it should be noted that the heat exchanger according to the invention might be inserted in oxygenation apparatuses or other mass transfer devices whose general structure is different from the one described, and that the heat exchanger may also be used as an independent unit.

In the practical embodiment of the invention, all the details may be replaced with other technically equivalent elements. Also, the materials employed, as well as the shapes and dimensions, may vary.

Where technical features mentioned in any claim are followed by reference signs, such reference signs have been included for the sole purpose of increasing the intelligibility of the claims and accordingly such reference signs do not have any limiting effect on the scope of each element identified by way of example by such reference signs.

## Claims

1. A device for heat exchange comprising:
-- a cylindrical shell (3) having a corrugated interior surface;
-- a core (4) having a corrugated inner and corrugated outer surface, the core being coaxially aligned within the cylindrical shell (3), wherein the interior surface of the cylindrical shell (3) is opposed to the outer surface of the core to form a first annular chamber (5);
-- a manifold having a corrugated exterior surface, the manifold being coaxially aligned within the core (4), wherein the exterior surface of the manifold (2) is opposed to the inner surface of the core to form a second annular chamber (19);
-- a first fluid inlet means (9) for introducing a first fluid into the first annular chamber (5)and a first fluid outlet means (10) for discharging the first fluid from the first annular chamber (5), wherein the first fluid inlet means (9) and first fluid outlet means (10) are opposed axially at opposite ends of the first annular chamber for passing the first fluid axially through the first annular chamber (5);
-- a second fluid inlet means (21, 22, 23) for introducing a second fluid for heat exchange into the second annular chamber (19) and a second fluid outlet means (24, 25, 26)for discharging the second fluid from the second annular chamber (19), wherein the second fluid inlet means and second fluid outlet means (24, 25, 26) are opposed axially at opposite ends of the second annular chamber (19) for passing the second fluid axially through the second annular chamber (19), wherein heat exchange occurs across the core (4) between the first and second fluid.

2. The device of claim 1, characterized in that the corrugated interior surface of the cylindrical shell(3), the inner and outer surfaces of the core (4), and the corrugated exterior surface of the manifold (2) each have helical corrugations (2a, 4a).

3. The device of claim 2, characterized in that the corrugated outer surface of the core (4)is discordant with the corrugated interior surface of the cylindrical shell (3).

4. The device of claim 2, characterized in that the corrugated inner surface of the core (4)is discordant with the corrugated exterior surface of the manifold (2).

5. The device of claim 2, characterized in that the corrugated outer surface of the core (4)is concordant with the corrugated interior surface of the cylindrical shell (3).

6. The device of claim 2, characterized in that the corrugated inner surface of the core (4) is concordant with the corrugated exterior surface of the manifold (2).

7. The device of claim 1, characterized in that the first fluid inlet means (9)and second fluid inlet means (21, 22, 23) are axially opposed at opposite ends of the core.

8. The device of claim 1, characterized in that the first fluid outlet means (10) and second fluid outlet means (24, 25, 26) are axially opposed at opposite ends of the core.

9. The device of claim 1, characterized in that the cylindrical shell (3) and corrugated core are integrally constructed to form a disposable module.

10. The device of claim 9, characterized in that the disposable module is removably coupled to the manifold (2).

11. The device of claim 1, characterized in that the outer periphery of at least one corrugation of the corrugated interior surface of the cylindrical shell (3) is in contact with the corrugated outer surface of the core (4), and the outer periphery of at least one corrugation of the corrugated exterior surface of the manifold (2) is in contact with the corrugated inner surface of the core (4).

12. The device of claim 1, characterized in that it further comprises a first and a second sealing gasket 6,7 for sealing a first and a second end, respectively, of the first annular chamber 5, and a third and a fourth sealing gasket 31a, 32a for sealing a first and a second end, respectively, of the second annular chamber (19).

13. The device of claim 1, characterized in that it further comprises a mass transfer means having an inner surface and an outer surface, the first fluid outlet means (24, 25, 25) being in fluid communication with the outer surface of the mass transfer means, and a third fluid inlet (16) and a third fluid outlet (18) in fluid communication with the inner surface of the mass transfer means; mass transfer occurs between the first fluid and a third fluid passing within the inner surface of the mass transfer means.

14. The device of claim 13, characterized in that the mass transfer means comprises a blood oxygenator, the first fluid inlet (9) is a blood inlet, the first fluid outlet (10) is a blood outlet, the second fluid inlet (21, 22, 23) is a water inlet, the second fluid outlet (24, 25, 26) is a water outlet, the third fluid inlet (16) is an oxygen inlet, and the third fluid outlet (18) is an oxygen outlet.

15. Use of a device according to claim 1 for heat exchange between a first fluid which is blood and a second fluid which is water.

## Patentansprüche

1. Vorrichtung zum Wärmeaustausch, **gekennzeichnet durch**
eine zylindrische Hülle (3) mit einer gerippten Innenfläche; ein Innengehäuse (4) mit einer gerippten Innen- und Außenfläche, wobei das Innengehäuse koaxial mit der zylindrischen Hülle (3) verbunden ist, und die Innenfläche der zylindrischen Hülle (3) der Außenfläche des Innengehäuses gegenüberliegt, um eine erste ringförmige Kammer (5) zu bilden;
ein Verteiler mit einer gerippten Außenfläche, wobei der Verteiler koaxial mit dem Innengehäuse (4) verbunden ist und die Außenfläche des Verteilers (2) der Innenfläche des Innengehäuses gegenüberliegt, um eine zweite ringförmige Kammer (19) zu bilden;
eine erste Fluideinlaßeinrichtung (9) zum Einlaß eines ersten Fluides in die erste ringförmige Kammer (5) sowie eine erste Fluidauslaßeinrichtung (10) zum Auslaß des ersten Fluides aus der ersten ringförmigen Kammer (5), wobei die erste Fluideinlaßeinrichtung (9) und die erste Fluidauslaßeinrichtung (10) an gegenüberliegenden Enden der ersten ringförmigen Kammer sich axial gegenüberliegen, um das erste Fluid axial durch die erste ringförmige Kammer (5) zu leiten;
eine zweite Fluideinlaßeinrichtung (21, 22, 23) zum Einlaß eines zweiten Fluides zwecks Wärmeaustausches in die zweite ringförmige Kammer (19) und eine zweite Fluidauslaßeinrichtung (24, 25, 26) zum Auslaß des zweiten Fluides aus der zweiten ringförmigen Kammer (19), wobei die zweite Fluideinlaßeinrichtung und die zweite Fluidauslaßeinrichtung (24, 25, 26) an gegenüberliegenden Enden der zweiten ringförmigen Kammer (19) sich axial gegenüberliegen, um das zweite Fluid axial durch die zweite ringförmige Kammer (19) zu leiten, wodurch ein Wärmeaustausch über das Innengehäuse (4) zwischen dem ersten und dem zweiten Fluid erfolgt.

2. Vorrichtung nach Anspruch 1, **dadurch gekennzeichnet, daß** die gerippte Innenfläche der gerippten zylindrischen Hülle (3), die Innen- und die Außenfläche des Innengehäuses (4) und die gerippte Außenfläche des Verteilers (2) gewindeförmige Rippen (2a, 4a) aufweisen.

3. Vorrichtung nach Anspruch 2, **dadurch gekennzeichnet, daß** sich die Rippung der Außenfläche des Innengehäuses (4) von der Rippung der Innenfläche der zylindrischen Hülle (3) unterscheidet.

4. Vorrichtung nach Anspruch 2, **dadurch gekennzeichnet, daß** sich die Rippung der Innenfläche des Innengehäuses (4) von der Rippung der Außenfläche des Verteilers (2) unterscheidet.

5. Vorrichtung nach Anspruch 2, **dadurch gekennzeichnet, daß** die Rippung der Außenfläche des Innengehäuses (4) mit der Rippung der Innenfläche der zylindrischen Hülle (3) abgestimmt ist.

6. Vorrichtung nach Anspruch 2, **dadurch gekennzeichnet, daß** die Rippung der Innenfläche des Innengehäuses (4) mit der Rippung der Außenfläche des Verteilers (2) abgestimmt ist.

7. Vorrichtung nach Anspruch 1, **dadurch gekennzeichnet, daß** die erste Fluideinlaßeinrichtung (9) und die zweite Fluideinlaßeinrichtung (21, 22, 23) an sich gegenüberliegenden Enden des Innengehäuses axial gegenüberliegend angeordnet sind.

8. Vorrichtung nach Anspruch 1, **dadurch gekennzeichnet, daß** die erste Fluidauslaßeinrichtung (10) und die zweite Fluidauslaßeinrichtung (24, 25, 26) an sich gegenüberliegenden Enden axial gegenüberliegend angeordnet sind.

9. Vorrichtung nach Anspruch 1, **dadurch gekennzeichnet, daß** die zylindrische Hülle (3) und das gerippte Innengehäuse integral geformt sind, um einen austauschbaren Modul zu bilden.

10. Vorrichtung nach Anspruch 9, **dadurch gekennzeichnet, daß** der austauschbare Modul mit dem Verteiler (2) lösbar verbunden ist.

11. Vorrichtung nach Anspruch 1, **dadurch gekennzeichnet, daß** der äußere Umfang von mindestens einer Rippung der gerippten Innenfläche der zylindrischen Hülle (3) sich in Kontakt mit der Rippung der Außenfläche des Innengehäuses (4) befindet, und der äußere Umfang von mindestens einer Rippung der gerippten Außenfläche des Verteilers (2) Kontakt zur Rippung der Innenfläche des Innengehäuses (4) hat.

12. Vorrichtung nach Anspruch 1, **dadurch gekennzeichnet, daß** diese weiterhin eine erste und eine zweite Dichtung (6, 7) zur Abdichtung von entsprechenden ersten und zweiten Enden der ersten ringförmigen Kammer (5) sowie eine dritte und eine vierte Dichtung (31a, 32a) zur Abdichtung von entsprechenden ersten und zweiten Enden der zweiten ringförmigen Kammer (19) umfaßt.

13. Vorrichtung nach Anspruch 1, **dadurch gekennzeichnet, daß** diese weiterhin eine Massenaustauscheinrichtung mit einer Innen- und einer Außenfläche besitzt, und daß die erste Fluidauslaßeinrichtung (24, 25, 26) in Fluidverbindung mit der Außenfläche der Massenaustauscheinrichtung, und ein dritter Fluideinlaß (16) sowie ein dritter Fluidauslaß (18) in Fluidverbindung mit der Innenfläche der Massenaustauscheinrichtung stehen; wobei der Massenaustausch zwischen dem ersten Fluid und einem dritten Fluid erfolgt, welches an der Innenfläche der Massenaustauscheinrichtung vorbeifließt.

14. Vorrichtung nach Anspruch 13, **dadurch gekennzeichnet, daß** die Massenaustauscheinrichtung eine Blutsauerstoffanreicherungseinrichtung umfaßt, und daß der erste Fluideinlaß (9) ein Bluteinlaß, der erste Fluidauslaß (10) ein Blutauslaß, der zweite Fluideinlaß (21, 22, 23) ein Wassereinlaß, der zweite Fluidauslaß (24, 25, 26) ein Wasserauslaß, der dritte Fluideinlaß (16) ein Sauerstoffeinlaß und der dritte Fluidauslaß (18) ein Sauerstoffauslaß ist.

15. Verwendung einer Vorrichtung nach Anspruch 1 zum Wärmeaustausch zwischen einem ersten Fluid, das aus Blut besteht, und einem zweiten Fluid, das aus Wasser besteht.

## Revendications

1. Dispositif pour l'échange de chaleur, comprenant :
- une coque cylindrique (3) ayant une surface intérieure cannelée ;
- un noyau (4) ayant une surface intérieure cannelée et une surface extérieure cannelée, le noyau étant coaxialement aligné à l'intérieur de la coque cylindrique (3), la surface intérieure de la coque cylindrique (3) étant opposée à la surface externe du noyau pour former une première chambre annulaire (5) ;
- une tubulure ayant une surface extérieure cannelée, la tubulure étant coaxialement alignée à l'intérieur du noyau (4), la surface extérieure de la tubulure (2) étant opposée à la surface interne du noyau pour former une seconde chambre annulaire (19) ;
- des premiers moyens d'entrée de fluide (9) pour introduire un premier fluide dans la première chambre annulaire (5) et des premiers moyens de sortie de fluide (10) pour évacuer le premier fluide de la première chambre annulaire (5), la première entrée de fluide (9) et les premiers moyens de sortie de fluide (10) étant opposés axialement à des extrémités opposées de la première chambre annulaire pour faire passer le premier fluide axialement à travers la première chambre annulaire (5) ;
- des seconds moyens d'entrée de fluide (21,22,23) pour introduire un second fluide pour l'échange de chaleur dans la seconde chambre annulaire (19) et des seconds moyens de sortie de fluide (24,25,26) pour évacuer le second fluide de la seconde chambre annulaire (19), les seconds moyens d'entrée de fluide et les seconds moyens de sortie de fluide (24,25,26) étant opposés axialement à des extrémités opposées de la seconde chambre annulaire (19) pour faire passer le second fluide axialement à travers la seconde chambre annulaire (19), l'échange thermique ayant lieu à travers le noyau (4) entre les premier et second fluides.

2. Dispositif selon la revendication 1,
caractérisé en ce que la surface intérieure cannelée de la coque cylindrique (3), les surfaces interne et externe du noyau (4), et la surface extérieure cannelée de la tubulure (2) présentent chacune des cannelures hélicoïdales (2a,4a).

3. Dispositif selon la revendication 2,
caractérisé en ce que la surface externe cannelée du noyau (4) est discordante avec la surface intérieure cannelée de la coque cylindrique (3).

4. Dispositif selon la revendication 2,
caractérisé en ce que la surface interne cannelée du noyau (4) est discordante avec la surface extérieure cannelée de la tubulure (2).

5. Dispositif selon la revendication 2,
caractérisé en ce que la surface externe cannelée du noyau (4) est concordante avec la surface intérieure cannelée de la coque cylindrique (3).

6. Dispositif selon la revendication 2,
caractérisé en ce que la surface interne cannelée du noyau (4) est concordante avec la surface extérieure cannelée de la tubulure (2).

7. Dispositif selon la revendication 1,
caractérisé en ce que las premiers moyens d'entrée de fluide (9) et les seconds moyens d'entrée de fluide (21,22,23) sont axialement opposés aux extrémités opposées du noyau.

8. Dispositif selon la revendication 1,
caractérisé en ce que les premiers moyens de sortie de fluide (10) et les seconds moyens de sortie de fluide (24,25,26) sont axialement opposés aux extrémités opposées du noyau.

9. Dispositif selon la revendication 1,
caractérisé en ce que la coque cylindrique (3) et le noyau cannelé sont construits en une pièce pour former un module jetable.

10. Dispositif selon la revendication 9,
caractérisé en ce que le module jetable est couplé de façon amovible à la tubulure (2).

11. Dispositif selon la revendication 1,
caractérisé en ce que la périphérie externe d'au moins une cannelure de la surface intérieure cannelée de la coque cylindrique (3) est en contact avec la surface externe cannelée du noyau (4), et la périphérie externe d'au moins une cannelure de la surface extérieure cannelée de la tubulure (2) est en contact avec la surface interne cannelée du noyau (4).

12. Dispositif selon la revendication 1,
caractérisé en ce qu'il comprend de plus des premier et second joints d'étanchéité (6,7) pour étanchéifier des première et seconde extrémités, respectivement, de la première chambre annulaire (5), et des troisième et quatrième joints d'étanchéité (31a,32a) pour étanchéifier des première et seconde extrémités, respectivement, de la seconde chambre annulaire (19).

13. Dispositif selon la revendication 1,
caractérisé en ce qu'il comprend de plus des moyens de transfert de masse ayant une surface interne et une surface externe, les premiers moyens de sortie de fluide (24,25,26) étant en communication fluidique avec la surface externe des moyens de transfert de masse, et une troisième entrée de fluide (16) et une troisième sortie de fluide (18) en communication fluidique avec la surface interne des moyens de transfert de masse, le transfert de masse ayant lieu entre le premier fluide et un troisième fluide passant à l'intérieur de la surface interne des moyens de transfert de masse.

14. Dispositif selon la revendication 13,
caractérisé en ce que les moyens de transfert de masse comprennent un oxygénateur de sang, la première entrée de fluide (9) est une entrée de sang, la première sortie de fluide (10) est une sortie de sang, la seconde entrée de fluide (21,22,23) est une entrée d'eau, la seconde sortie de fluide (24,25,26) est une sortie d'eau, la troisième entrée de fluide est une entrée d'oxygène, et la troisième sortie de fluide (18) est une sortie d'oxygène.

15. Utilisation d'un dispositif selon la revendication 1 pour l'échange thermique entre un premier fluide qui est du sang et un second fluide qui est de l'eau.
